# EUROPEAN PATENT SPECIFICATION

(11) **EP 3 104 863 B1**
(45) Date of publication and mention of the grant of the patent: **18.10.2023**
(21) Application number: 14792485.6
(22) Date of filing: 30.10.2014
(51) Int. Cl.: A61K 31/60, A61K 31/603, A61K 31/616, A61K 45/06, A61P 35/00

(54) **SALICYLATES FOR USE IN THE TREATMENT OF CANCER**
SALICYLATE ZUR VERWENDUNG IN DER BEHANDLUNG VON KREBS
SALICYLATES POUR L'UTILISATION DANS LE TRAITEMENT DU CANCER

(30) Priority: 07.11.2013 EP 13191964
(43) Date of publication of application: 21.12.2016
(73) Proprietor: Kreutz, Marina, 93426 Roding (DE); Trapp, Carolin, 79106 Freiburg (DE)
(72) Inventor: KREUTZ, Werner, 79219 Staufen (DE)
(74) Representative: Lederer & Keller Patentanwälte Partnerschaft mbB
(86) International application number: PCT/EP2014/073376
(87) International publication number: WO 2015/067526

(56) References cited:
- WO-A1-2005/016354
- US-A- 5 985 927
- US-B1- 6 395 720
- FISCHER BIANCA ET AL: "Acidic pH inhibits non-MHC-restricted killer cell functions", CLINICAL IMMUNOLOGY, ACADEMIC PRESS, US, vol. 96, no. 3, 1 September 2000 (2000-09-01), pages 252-263, XP009175007, ISSN: 1521-6616
- ELWOOD P C ET AL: "Aspirin, salicylates, and cancer", THE LANCET, LANCET LIMITED. LONDON, GB, vol. 373, no. 9671, 11 April 2009 (2009-04-11), pages 1301-1309, XP026037890, ISSN: 0140-6736, DOI: 10.1016/S0140-6736(09)60243-9 [retrieved on 2009-04-09]

## Description

### Field of invention

The present invention relates to salicylates for use in the treatment of cancer comprising a certain dosage regimen.

### Background of the invention

Salicylates acetylsalicylic acid (INN: ASA), 4-aminosalicylic acid (commonly known as p-aminosalicylic acid, PAS) and 5-[2,4-difluorophenyl]-salicylic acid (INN: Diflunisal) are well known in the art and will be referred to as ASA, PAS and Diflunisal (sometimes abbreviated as Diflu), respectively, from here on in.

WO 96/30003 proposes to use salicylates and salicylate derivatives for the control of tumor tissue which at pH of less than 7 are protonated or release a substance the protonated compound or the released substance having a more strongly destructive effect on cells than the unprotonated compound or the compound before release of the substance. WO 96/30003 also generally discloses mixtures of two or more compounds.

WO 98/58639 relates to the selective attack of cancer cells existing in an acidic extra-cellular environment by synergistically acting combinations of salicylates, such as Diflunisal-ASA, Diflunisal-PAS or ASA-PAS. However, there is still a need for an improved dosage regimen that demonstrates excelled anti-tumor effects in patients.

WO 2005/016354 offers a possibility for targeted attack on basic tumor fractions was offered by applying Diflunisal solo. But Diflunisal solo attack is restricted to acidic and basic tumor fractions existing in situations of low albumin levels. In organs with high albumin levels of about 0.4-0.5 mM, such as liver, the Diflunisal solo activity fails due to low loading of Diflunisal onto albumin.

US 5,985,927 discloses a medicinal product for the selective control of tumor tissue comprising at least two different compounds having a pH below 7 when the compounds are present in a protonated form. The compounds are selected from certain benzoic acid derivatives.

US 6,395,720 relates to a method of treating carcinomatous disorders, comprising administering to a patient in need thereof a pharmaceutically effective amount of a synergistic combination of 2-hydroxy-4-aminobenzoic acid and acetylsalicylic acid.

P. C. Elwood et al., describe in Lancet, 2009; 373: 1301-09 that individuals taking aspirin and related non-steroidal anti-inflammatory drugs have reduced risk of large bowel cancer.

A further problem is that tumors and metastases are not attacked by the immune system under physiological conditions, i.e. that they co-exist with normal tissues.

As far as tumor fractions exist in acidic milieu the resistance against immune attack can be explained by an archaic principle realized by acidification of the extra-cellular environment. As it was investigated, the pH-dependence of immune killing actions, it turned out that all the four known kill actions of the immune system are pH dependent and that they cease to occur at pH < 6.9. So a simple way to create blockage of the immune system is to produce acidification in the extracellular environment of cancer cells. (Kreutz et al., Science, 1980; 210: 1253-1255. Kreutz et al., Tumor Biol., 1994; 15: 304-310. Kreutz et al., Immunology, 2000; 99: 375-384. Kreutz et al., J. Immunother., 2000; 23(2): 196-207. and Kreutz et al., Clinical Immunology, 2000; 96(3): 252-263.)

These facts suggest that tumor fractions existing in basic milieu should be attacked by the immune system. However, those activities are not observed; therefore, there must exist a further defense system (protection factor) installed in the cancer cells.

It would therefore be desirable to eliminate this "protection factor" in the cancer cells.

It is therefore an object of the present invention to solve the above problems, in particular to provide a more effective treatment of cancer.

It has now surprisingly been found that the above problems can be solved by the administration of ASA, PAS and Diflunisal using a certain dosage regimen.

### Summary of the invention

The present invention thus relates to salicylates for use in the treatment of cancer comprising the following dosage regimen:
a) administering to a patient an initial dose of either acetylsalicylic acid (ASA) or 5-[2,4-difluorophenyl]-salicylic acid (Diflunisal) on a first day of treatment,
b) administering to the patient a second dose of at least two different salicylates selected from: acetylsalicylic acid (ASA), 4-aminosalicylic acid (PAS) and 5-[2,4-difluorophenyl]-salicylic acid (Diflunisal) on a next day of treatment, wherein the dose of acetylsalicylic acid does not exceed 80 mg/kg bodyweight of the patient in step b) when acetylsalicylic acid and 5-[2,4-difluorophenyl]-salicylic acid are administered together.

The term "first Day of treatment" as used herein describes 12 to 24 hours, preferably 24 hours of treatment, which consists of the administration of an initial dose of either ASA or Diflunisal described in step a). It is to be understood that the dosage regimen of the present invention starts with the first Day of treatment. There can be, however, further administration of the same or other active agents prior to the first Day of treatment.

The term "next Day of treatment" as used herein describes 24 hours of treatment that comes after the first Day of treatment or any later Day of treatment. The next Day of treatment is preferred to be consecutive from the first Day of treatment or any later Day of treatment. According to the present invention, it is preferred that the second dose of at least two different salicylates of step b) is administered to a patient on each of at least three consecutive Days of treatments, such as at least six consecutive Days of treatment. Most preferably the second dose of at least two different salicylates of step b) is administered to a patient on each of four consecutive Days of treatment.

In a preferred embodiment, the initial dose of ASA in step a) is between 40-70 mg/kg, more preferably 50-70 mg/kg and most preferably 50-60 mg/kg of bodyweight of the patient. As used herein mg/kg means mg/kg bodyweight of the patient.

In another embodiment of the present invention, it is preferred that the initial dose of Diflunisal is between 20-60 mg/kg, more preferably 25-50 mg/kg and most preferably 35-45 mg/kg bodyweight of the patient.

After administering to the patient the initial dose of either ASA or Diflunisal on the first Day of treatment, it is preferred that a dose of ASA in step b) is between 30-120 mg/kg, more preferably 40-120 mg/kg and most preferably 60-110 mg/kg bodyweight of the patient.

After administering to the patient the initial dose of either ASA or Diflunisal on the first Day of treatment, it is preferred that the dose of PAS in step b) is between 100-300 mg/kg, more preferably 150-250 mg/kg bodyweight of the patient.

After administering to the patient the initial dose of either ASA or Diflunisal on the first Day of treatment, it is preferred that the dose of Diflunisal in step b) is between 20-60 mg/kg, more preferably 30-45 mg/kg bodyweight of the patient.

Proliferation of cancer cells should demand additional energy supply besides the energy production of the mitochondrial respiratory chain. This additional supplied energy should be guaranteed by glycolysis. As a consequence lactate export of the cancer cells should be observed.

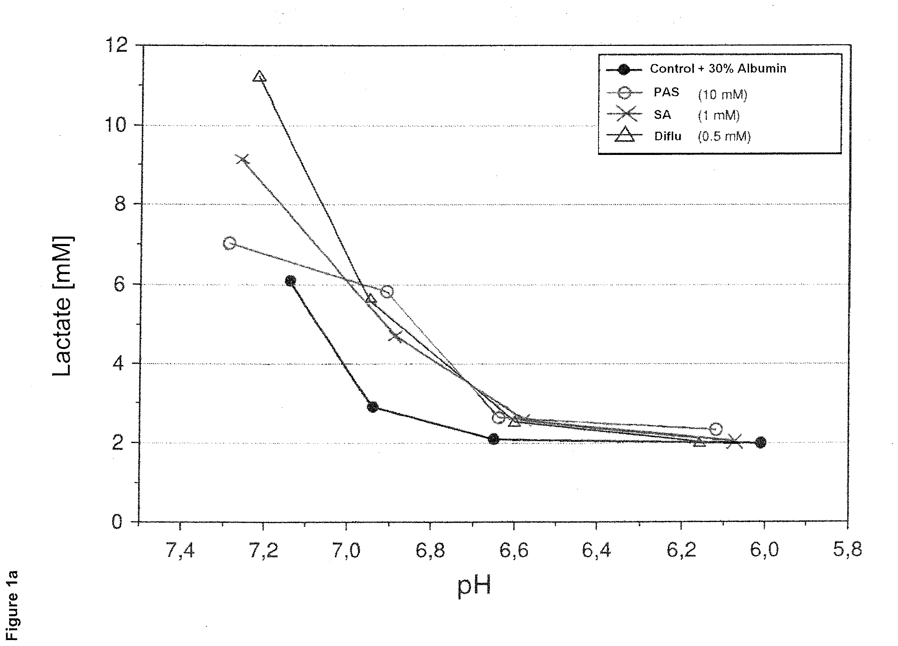

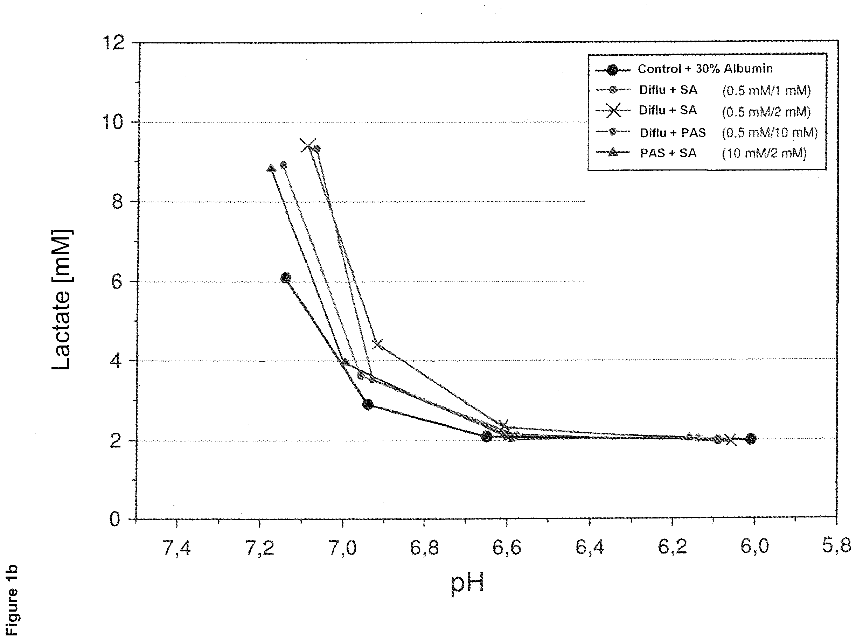

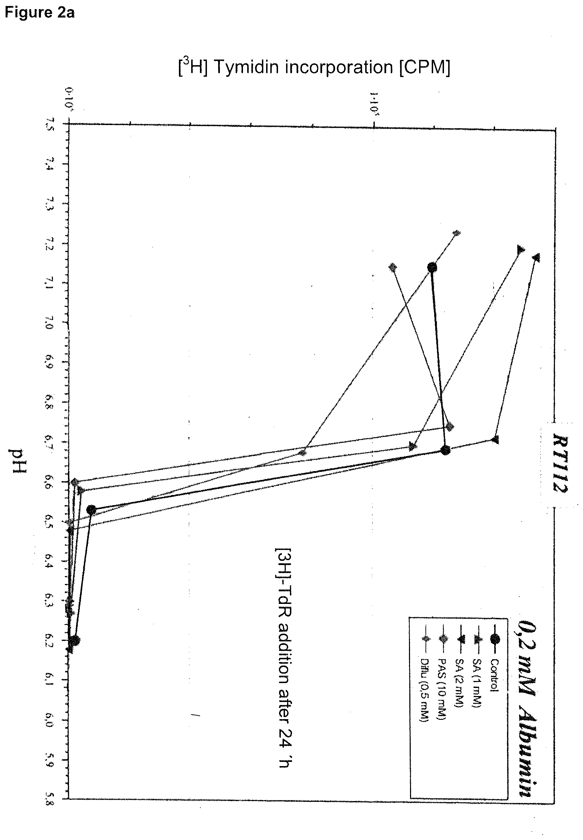

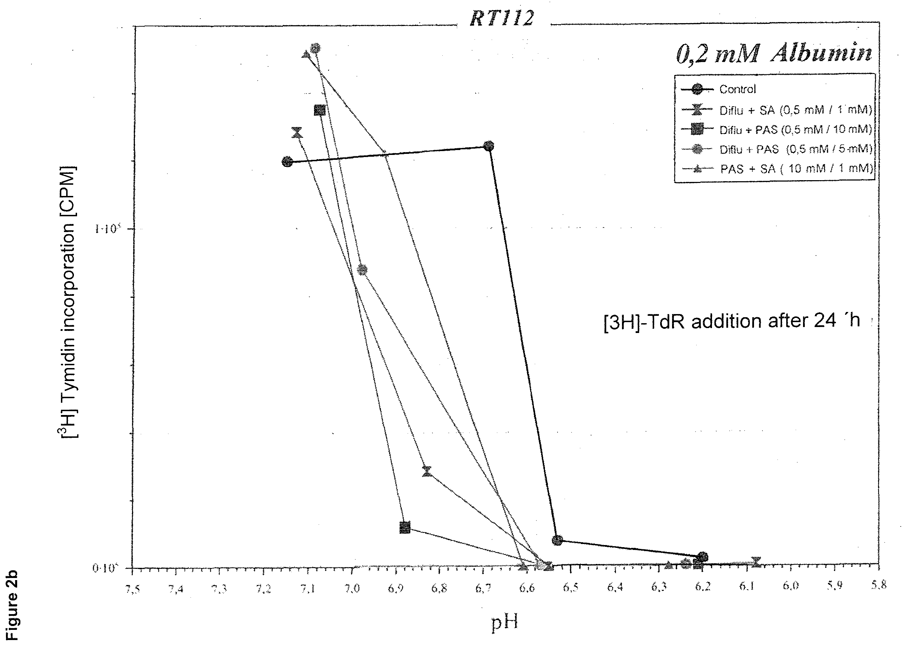

This inter-correlation can be demonstrated by measurements of the lactate export and in parallel by induced proliferation measurements on isolated cancer cells of solid tumors. The result of such experiments are demonstrated in Fig. 1 and 2 in case of a bladder cell line RT 112. The induction of lactate export and of proliferation is performed with administration of Diflunisal, with ASA and their combinations. Administration of Diflunisal or ASA alone on the first Day of treatment produce stimulation of lactate export all over the pH range, however, their combinations only stimulate at basic pH. This different behavior is due to pore formation, i.e. cell kill at acidic pH by the combinations. These lactate export measurements should correlate with the stimulation of proliferation. The proliferation tests carried out under the same conditions as performed with lactate export measurements confirm the supposed concept (Fig. 2). Proliferation of the stimulated cancer cells shows the same enhancement effect as is observed in case of lactate export.

According to the present invention, it is shown that those proliferated cells, after stimulation with the initial dose of ASA or Diflunisal on the first Day of treatment, become attacked by the immune system. Such tests cannot be performed with nude mice that are immune deficient, but only by patient tests.

The patient tests were conceived such that on the first Day of treatment either Diflunisal or ASA were administered and on the next at least three consecutive Days of treatment at least two different salicylates of step b), preferably combinations of Diflunisal-ASA or ASA-PAS, are administered for elimination of the acidic proliferated cancer cells.

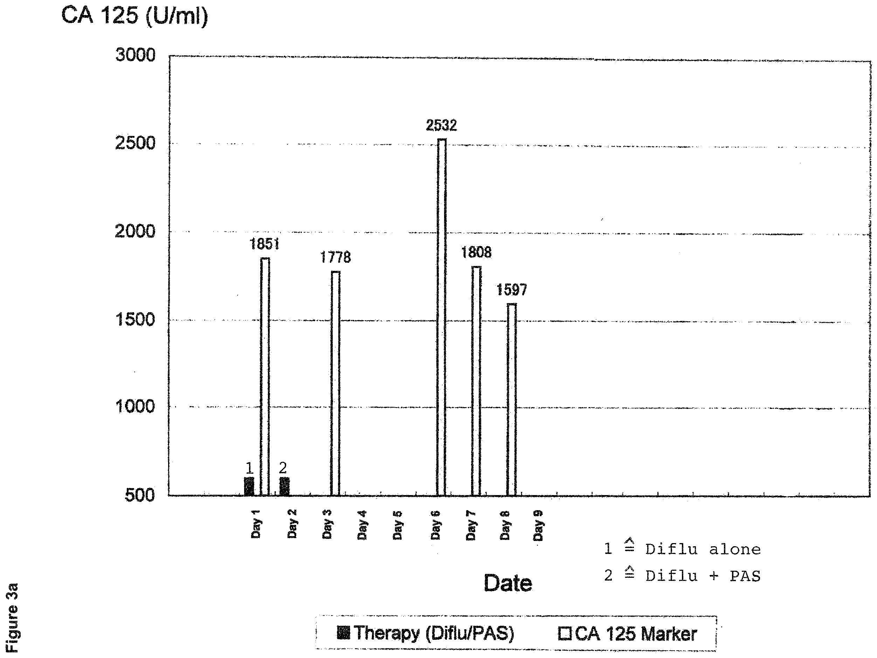

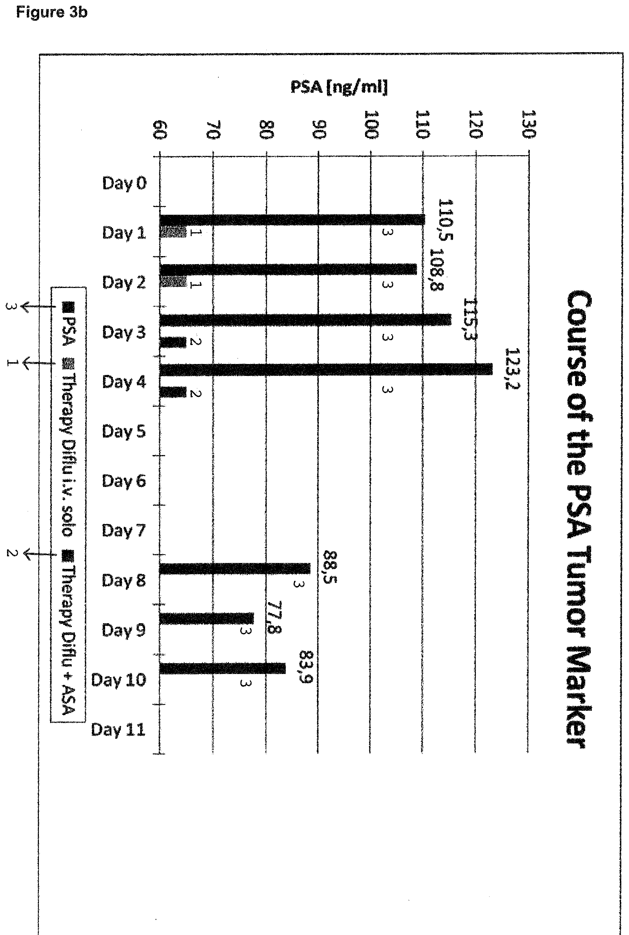

In Fig. 3a and 3b the results of two of such patient tests are presented. In Fig. 3a the test was performed in case of ovarian cancer and in Fig. 3b in case of a prostate cancer. A typical feature of such proliferation tests is that on the third day after stimulation (i.e. after first Day of treatment) the proliferation starts to occur. On the fifth and sixth day after proliferation (i.e. five and six consecutive Days of treatment after the first Day of treatment) stimulation of the immune system attacks the proliferated cells. This is a typical behavior of cT-lymphocytes activity well known in immunology. This attack, however, should be restricted to cancer cells, which have been proliferated in basic milieu. Acidic proliferated cancer cells aren't bothered by the immune system as already mentioned. This is the reason why in cancer therapy strategies, already after the first stimulation day (i.e. first Day of treatment) on the second, third and the fourth Day of treatment at least two different salicylates of step b) have to be administered in order to destroy the formed acidic cancer cell fractions, as has been done with the demonstrated therapy examples.

In the present invention, the dose of ASA does not exceed 80 mg/kg in step b) when ASA and Diflunisal are administered as at least two different salicylates of step b), in order to become active in acidic milieu. High ASA dosages will prevent Diflunisal binding onto the cancer cell membrane.

It is to be understood that the initial dose of ASA or Diflunisal that is administered on the first Day of treatment can be administered in fractions, spread out over first Day of treatment, wherein the total amount of ASA or Diflunisal can be administered as one individual bolus dose, or for example two, three, four or five fractions of the initial dose throughout the first Day of treatment. The time between each separate fraction dose of ASA or Diflunisal on the first Day of treatment can vary between 1-8 hours, more preferably 1-6 hours, and most preferably is about 6 hours.

In one embodiment when Diflunisal is administered as the initial dose of step a) it is preferred that Diflunisal is administered in two separate fractions as a first fraction dose of between 20-35 mg/kg bodyweight of the patient and then after 4-8 hours as a second fraction dose of 12-20 mg/kg bodyweight of the patient, more preferably as a first fraction dose of between 25-35 mg/kg bodyweight of the patient and then after about 6 hours as a second fraction dose of about 15 mg/kg bodyweight of the patient.

At least two different salicylates of step b) can be administered in fractions, together, separate and/or spread out over the Day of treatment. It is to be understood that, Diflunisal, ASA and PAS combinations can be administered as one whole total dose together at one specific time or said combinations can be spread out individually or in combinations throughout the Day of treatment. These doses can also be separated in fraction doses, such as two, three, four, five or six fraction doses of Diflunisal, ASA and PAS either in combinations or individually. According to the present invention there is an unlimited amount of different combinations of doses, fractions of doses and time between the doses of administration of at least two different salicylates of step b).

In another embodiment a time between last administration of a dose on any Day of treatment and the first administration of a dose on next Day of treatment is at least 12 hours, and preferably not more than 24 hours.

In another embodiment a treatment cycle can be characterized by: administration of Diflunisal in step a) is repeated for four consecutive Days of treatment and administration of the at least two different salicylates of step b) is repeated for at least two consecutive Days of treatment. It is preferred that the treatment cycle is repeated at least three times, such as five times. Most preferably the treatment cycle is repeated four times.

This possibility to get the basic tumor fractions eliminated by the activated immune system under controlled conditions open up the chance to attack tumors to complete remission. The activation of the immune system in reality means that the "protection factor" established in the cancer cells against immune attack is eliminated, which indicates that by proliferation in basic environment the cancer cells lose their protection factor.

But in the course of tests it became apparent how to artificially activate the immune system to be conditioned for killing cancer cells in basic environment. The "protection factor" also can be eliminated by the administration of high ASA doses of between 90-120 mg/kg bodyweight of the patient. Moreover, not only the protection factor became eliminated but also the proliferation was stopped.

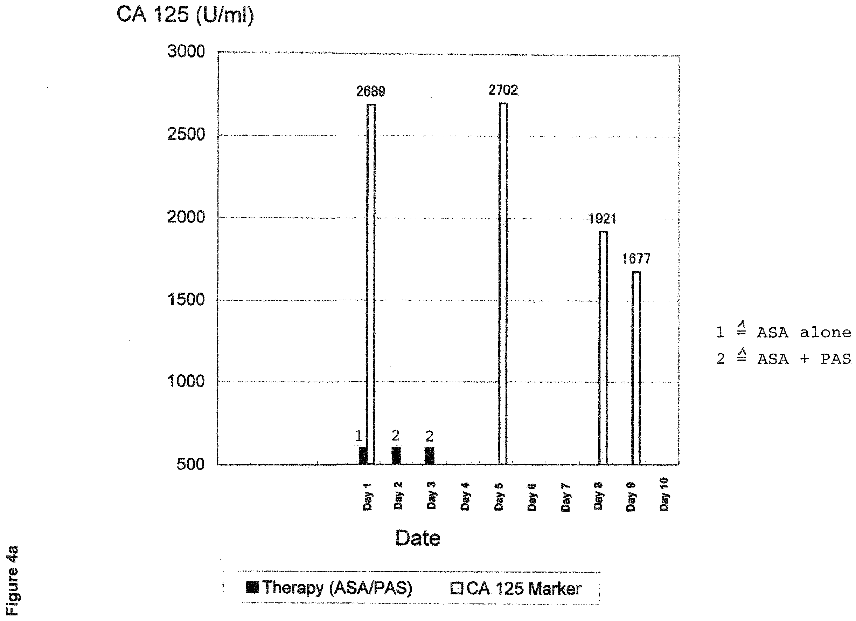

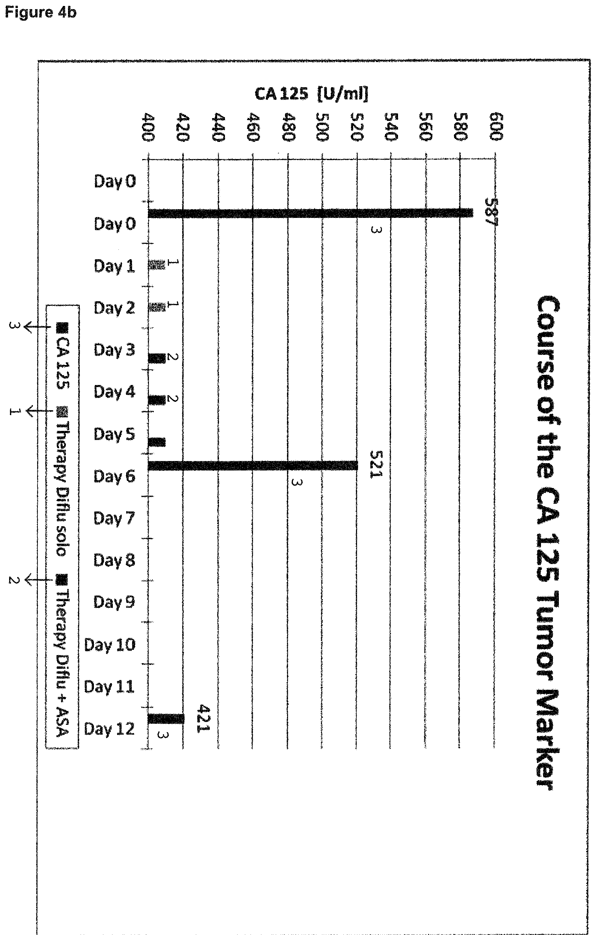

This result means, that by administrating at least two different salicylates of step b), preferably ASA-Diflunisal or ASA-PAS combinations, in case the ASA doses are increased up to 90-120 mg/kg bodyweight of the patient, proliferations are stopped and after the fifth and sixth day (after the initial dose of ASA is administered on the first Day of treatment), the immune system starts to kill the cancer cells freed from the protection factor. The results of two of such test examples in case of ovarian and prostate cancer are presented in Fig. 4a and 4b. The administered doses that provide this effect are between 90-120 mg/kg bodyweight of the patient of ASA.

All types of tumors and cancer cells in the acidic and basic milieu thus can be killed with at least two different salicylates of step b), preferably Diflunisal-ASA or ASA-PAS combinations.

For establishing routine protocols two ways of inducing proliferation in tumors and metastases exist: preferably by administering the initial dose of ASA between 40-60 mg/kg bodyweight of the patient or administering the initial dose of Diflunisal in one or two fraction doses, wherein the first fraction dose is at low concentrations of 25-30 mg/kg bodyweight of the patient and after about 6 hours with a second fraction dose of about 15 mg/kg bodyweight of the patient. After this initial dose on the first Day of treatment, three consecutive Days of therapy should follow by administration of at least two different salicylates of step b), preferably ASA-Diflunisal combinations in order to eliminate acidic tumor fractions. In one embodiment the initial dose on the first Day of treatment may be ASA followed by administration of at least two different salicylates of step b), preferably ASA-PAS combination, for two consecutive Days of treatment, more preferably at least three consecutive Days of treatment.

It is believed that the administration of ASA alone generally is not successful in combating cancer cells. As already outlined administration of the initial dose of ASA on the first Day of treatment stimulates proliferation of cancer cells in acidic as well as in basic milieu. The produced acidic cancer cells cannot be attacked by the immune system afterwards, i.e. in consequence tumors would start to grow in acidic milieu. Therefore as another embodiment of the present invention, if the initial dose of ASA on first Day of treatment is administered (as stimulant) followed by three consecutive Days of treatment of at least two different salicylates of step b), preferably ASA-Diflunisal, Diflunisal-PAS or ASA-PAS combinations, which are administered for the elimination of the acidic tumor fractions.

According to the criteria outlined before, including the use of the immune system activation under special conditions, it has opened a possibility to attack tumors to complete remission either by administering an initial dose of ASA on the first Day of treatment, preferably as one individual bolus dose, followed by administration of at least two different salicylates of step b), preferably ASA-PAS combinations; or by administering initial dose of Dilfunisal on the first Day of therapy, preferably as one individual bolus dose, followed by administration of at least two different salicylates of step b), preferably Diflunisal-ASA combinations.

### Detailed description of the invention

The present invention is described in more detail in the figures and examples.

**Figures:**
- Fig. 1 a/b:: Measurements of the lactate-transport (export) with the bladder cancer cell line RT 112 in the presence of 0.2 mM albumin (see material and methods). There is plotted a control measurement, which indicates lactate efflux is pH dependent. The lactate efflux changes when ASA, PAS, Diflunisal (1a) and Diflunisal combinations with PAS and ASA (1b) are added. ASA, PAS and Diflunisal administered alone accelerate the lactate efflux all over the pH range from pH 6.5 to 7.4. Combinations of Diflunisal-ASA or Diflunisal-PAS also produce acceleration but only at pH > 7.0 compared to the Control.
- Fig. 2a/b:: Proliferation measurements by incorporation of 3H-thymidine into the DNA of RT 112 cancer cells (see material and methods). Control proliferation means proliferation without attack of toxic compounds. The experiments were carried out in the presence of 0.2 mM albumin. ASA at 1 mM and 2 mM shows appreciable enhancement of proliferation all over the proliferation range of pH 6.5-7.4 (2a). Diflunisal inhibits proliferation slightly up to pH 7.1 because of weak pore-formation, however, above pH 7.1 it produces enhancement, as well (2a). Combinations of Diflunisal with ASA or PAS show complete proliferation stop up to 7.0 (2b). ASA-PAS combinations enhance proliferation above pH 6.8 (2b).
- Fig. 3a:: Individual patient test with an ovarian carcinoma to demonstrate proliferation stimulation followed by immune response, by administration of two Days of treatment with Diflunisal-PAS combinations. The test started with an oral 33 mg/kg Diflunisal dosage in the evening, followed by a further oral 33 mg/kg Diflunisal dose the next Day of treatment in parallel with 300 mg/kg PAS was administered. The main proliferation is stimulated by the Diflunisal-PAS combination at the second Day of treatment. Proliferation occurs on the fifth and sixth Day of treatment followed by immune response on the seventh and eighth Day of treatment.
- Fig. 3b:: Individual patient test with a prostate cancer. The proliferation induction is caused by the Diflunisal dosage of 25 mg/kg on the first Day of treatment. On the two consecutive Days of treatment combinations of Diflunisal-ASA at 25 mg/kg, respectively 60 mg/kg are administered to eliminate acidic tumor fraction. The proliferation starts on Day three of treatment. The immune attack by cT-lymphocytes occurs on Day five and Day six of treatment. Registered is the course of the PSA marker of the patient.
- Fig. 4a:: Individual patient test with an ovarian carcinoma by administration of initial dose of ASA (solo as bolus) and administration of ASA-PAS combination on the two consecutive Days of treatment. In this case no stimulation effect is observed but a strong immune response after five Days of treatment. A proliferation phase in this assay is conceived to be cancelled by the high ASA dosage of >90 mg/kg. The course of CA 125 marker was registered as indication of activity.
- Fig. 4b:: Individual patient test with an ovarian cancer with Diflunisal-ASA administration. On the first Day of treatment an initial dose of Diflunisal oral dosage of 30 mg/kg is administered. On the second up to the fifth Day of treatment Diflunisal-ASA combinations were administered at oral doses of 30 mg/kg and 60 mg/kg respectively. On the fourth Day of treatment the ASA dose increases to >90 mg/kg by daily accumulation and continues to increase on the fifth Day of treatment. After the fifth Day of treatment, the immune system starts to become active up to the twelfth Day of treatment. During the five Days of treatment weak acidic action by Diflunisal-ASA is observed. It is registered the CA 125 marker course of the patient.

### Examples

### Materials and Methods :

### Culture of tumor cells:

The human bladder tumor cell line RT112 or the human chronic myeloid leukemia cell line K562 were purchased from DKFZ Tumorzell- und datenbank (Institut für experimentelle Pathologie, Heidelberg, FRG) or DSM (Deutsche Sammlung von Mikroorganismen und Zellkulturen GmbH, Braunschweig, FRG) supplemented with 2 mM L-glutamine (BIOCHROM, Berlin, FRG), 1% NEAE (BIOCHROM), 10% heat-inactivated fetal calf serum (BIOCHROM), 100 U/ml penicillin and 50 µg/ml Streptomycin (BIOCHROM) at 37 oc and 5 % co2. Only mycoplasma-free cell cultures were used; this was frequently tested using a specific detection kit (BOEHRINGER MANNHEIM, Mannheim, FRG).

### Lactate transport:

### Qualitative, enzymatic determination of lactate in supernatants of RT112 cells at 340 nm

RT112 cells were harvested, washed twice and re-suspended in culture media to yield a density of 2 × 105/ml. 500 µl/well of the RT112 cell suspension were seeded into 24 well microtiter plates (BD) and incubated over night at 37 °C and 5 % C~. The Supernatant was replaced by 450 µl pH-adjusted culture medium (pH 6.0- 7.4) and 50 111 of the solution of preferred substances or 500 111/well pH-adjusted culture medium alone (control). Again, the cells were incubated for 24 hat 37 °C and 5% C02. The measurement of lactate in the supernatants at 340 nm (PERKIN-ELMER, Lambda 17. UV-VIS Spectrophotometer, Überlingen, FRG) was performed according to the manufacturer's instructions (SIGMA DIAGNOSTICS, Deisenhofen, FRG; Lactate: Quantitative, Enzymatic Determination of Lactate in Whole Blood at 340 nm (Procedure No. 826-UV)).

### Proliferation:

### [³H]-TdR incorporation assay

The influence of cytotoxic molecules on the proliferation ofRT112 tumor cells was quantified by the measurement of the incorporated tritiated thymidine ([³H]-TdR) into the cellular DNA of the tumor cells by liquid scintillation counting (LSC). In brief, RT112 cells are harvested, washed twice and re-suspended in culture media (2.5 × 105 /ml). 100 µl/well of the RT112 cell suspension were seeded into 96 well microtiter plates (BECTON DICKINSON) and incubated over night at 37 °C and 5% CO₂. The supernatant was replaced by 90 µl pH-adjusted culture medium (pH 6.0- 7.4) and 10 µl of the solution of preferred substances or 10 µl/well pH-adjusted culture medium alone (control). The cells were incubated for various time periods at 37°C and 5 % CO₂. Thereafter, cells were washed twice and re-incubated in fresh pH-adjusted culture medium. And pulsed for 24 h by the addition of 23.125 kBq [³H]-TdR/well (925 kBq/ml, AMERSHAM, Braunschweig, FRG). After freezing and thawing, radioactive DNA of the cultures were transferred to glass fiber filters with an automatic cell harvester (PHARMACIA). The incorporated radioactivity was measured in a liquid scintillation counter (PHARMACIA).

### Apoptosis:

### Determination of cytoplasmic histone-associated-DNA-fragments (mono- and oligonucleosomes) after induced cell death

The qualitative and quantitative *in vitro* determination of apoptosis in RT112 tumor cells was analysed with a photometric enzyme-immunoassay. In brief, RT112 cells were harvested, washed twice and re-suspended in culture media to yield a density of 2.5 × 10⁵/ml. 100 µl/well of the RT112 cell suspension were seeded into 96 well microtiter plates (BECTON DICKINSON) and incubated over night at 37°C and 5 % CO₂. The supernatant was replaced by 90 µl pH-adjusted culture medium (pH 6.0- 7.4) and 10 µl of preferred substances or 100 µl/well pH-adjusted culture medium alone (control). Again, the cells were incubated for various time periods at 37°C and 5 % CO₂. Supernatants of the test cultures were discarded and RT 112 cells were lysed in lysis buffer for 30 min at RT. The cytoplasmic fraction was transferred into a streptavidin-coated microtiter plate and the working procedure for the ELISA was performed according to the manufacturer's instructions (BOEHRINGER MANNHEIM).

## Claims

1. Salicylates for use in the treatment of cancer comprising the following dosage regimen:
a) administering to a patient an initial dose of either acetylsalicylic acid or 5-[2,4-difluorophenyl]-salicylic acid on a first Day of treatment,
b) administering to the patient a second dose of at least two different salicylates selected from: acetylsalicylic acid, 4-aminosalicylic acid and 5-[2,4-difluorophenyl]-salicylic acid on a next Day of treatment, wherein the dose of acetylsalicylic acid does not exceed 80 mg/kg bodyweight of the patient in step b) when acetylsalicylic acid and 5-[2,4-difluorophenyl]-salicylic acid are administered together.

2. Salicylates for use in the treatment of cancer comprising the dosage regimen according to claim 1, wherein the second dose is administered to a patient on each of at least three consecutive Days of treatment.

3. Salicylates for use in the treatment of cancer comprising the dosage regimen according to any of preceding claims, wherein the initial dose of acetylsalicylic acid in step a) is between 40-70 mg/kg bodyweight of the patient.

4. Salicylates for use in the treatment of cancer comprising the dosage regimen according to any of preceding claims, wherein the initial dose of 5-[2,4-difluorophenyl]-salicylic acid in step a) is between 20-60 mg/kg bodyweight of the patient.

5. Salicylates for use in the treatment of cancer comprising the dosage regimen according to any of preceding claims, wherein the dose of acetylsalicylic acid in step b) is between 30-120 mg/kg bodyweight of the patient.

6. Salicylates for use in the treatment of cancer comprising the dosage regimen according to any of preceding claims, wherein the dose of 4-aminosalicylic acid in step b) is between 100-300 mg/kg bodyweight of the patient.

7. Salicylates for use in the treatment of cancer comprising the dosage regimen according to any of preceding claims, wherein the dose of 5-[2,4-difluorophenyl]-salicylic acid in step b) is between 20-60 mg/kg bodyweight of the patient.

8. Salicylates for use in the treatment of cancer comprising the dosage regimen according to any of preceding claims, wherein the initial dose is administered in fractions spread out over first Day of treatment.

9. Salicylates for use in the treatment of cancer comprising the dosage regimen according to claim 8, wherein the initial dose of 5-[2,4-difluorophenyl]-salicylic acid in step a) is administered in two separate fractions as a first fraction dose of between 20-35 mg/kg bodyweight of the patient and then after 4-8 hours as a second fraction dose of 12-20 mg/kg bodyweight of the patient.

10. Salicylates for use in the treatment of cancer comprising the dosage regimen according to any of preceding claims, wherein the at least two different salicylates in step b) are administered in fractions, together, separate and/or spread out over the Day of treatment.

11. Salicylates for use in the treatment of cancer comprising the dosage regimen according to any of preceding claims, wherein a time between last administration of a dose on any Day of treatment and the first administration of a dose on next Day of treatment is at least 12 hours.

12. Salicylates for use in the treatment of cancer comprising the dosage regimen according to any of claims 1, 4 to 11, wherein a treatment cycle is **characterized by**: administration of 5-[2,4-difluorophenyl]-salicylic acid in step a) is repeated for four consecutive Days of treatment and administration of the at least two different salicylates of step b) is repeated for at least two consecutive Days of treatment.

13. Salicylates for use in the treatment of cancer comprising the dosage regimen according to claim 12, wherein the treatment cycle is repeated at least 3 times.

## Patentansprüche

1. Salicylate zur Verwendung bei der Behandlung von Krebs, umfassend den folgenden Dosierungsplan:
a) Verabreichen einer Startdosis von entweder Acetylsalicylsäure oder 5-[2,4-Difluorphenyl]-salicylsäure am ersten Tag der Behandlung an einen Patienten,
b) Verabreichen einer zweiten Dosis von mindestens zwei verschiedenen Salicylaten, ausgewählt aus: Acetylsalicylsäure, 4-Aminosalicylsäure und 5-[2,4-Difluorphenyl]-salicylsäure, am nächsten Tag der Behandlung an den Patienten, wobei die Dosis von Acetylsalicylsäure 80 mg/kg Körpergewicht des Patienten in Schritt b) nicht überschreitet, wenn Acetylsalicylsäure und 5-[2,4-Difluorphenyl]-salicylsäure zusammen verabreicht werden.

2. Salicylate zur Verwendung bei der Behandlung von Krebs, umfassend den Dosierungsplan nach Anspruch 1, wobei die zweite Dosis dem Patienten an jedem von mindestens drei aufeinanderfolgenden Tagen der Behandlung verabreicht wird.

3. Salicylate zur Verwendung bei der Behandlung von Krebs, umfassend den Dosierungsplan nach einem der vorstehenden Ansprüche, wobei die Startdosis von Acetylsalicylsäure in Schritt a) zwischen 40 und 70 mg/kg Körpergewicht des Patienten liegt.

4. Salicylate zur Verwendung bei der Behandlung von Krebs, umfassend den Dosierungsplan nach einem der vorstehenden Ansprüche, wobei die Startdosis von 5-[2,4-Difluorphenyl]-salicylsäure in Schritt a) zwischen 20 und 60 mg/kg Körpergewicht des Patienten liegt.

5. Salicylate zur Verwendung bei der Behandlung von Krebs, umfassend den Dosierungsplan nach einem der vorstehenden Ansprüche, wobei die Dosis von Acetylsalicylsäure in Schritt b) zwischen 30 und 120 mg/kg Körpergewicht des Patienten liegt.

6. Salicylate zur Verwendung bei der Behandlung von Krebs, umfassend den Dosierungsplan nach einem der vorstehenden Ansprüche, wobei die Dosis von 4-Aminosalicylsäure in Schritt b) zwischen 100 und 300 mg/kg Körpergewicht des Patienten liegt.

7. Salicylate zur Verwendung bei der Behandlung von Krebs, umfassend den Dosierungsplan nach einem der vorstehenden Ansprüche, wobei die Dosis von 5-[2,4-Difluorphenyl]-salicylsäure in Schritt b) zwischen 20 und 60 mg/kg Körpergewicht des Patienten liegt.

8. Salicylate zur Verwendung bei der Behandlung von Krebs, umfassend den Dosierungsplan nach einem der vorstehenden Ansprüche, wobei die Startdosis in über den ersten Tag der Behandlung verteilten Teilen verabreicht wird.

9. Salicylate zur Verwendung bei der Behandlung von Krebs, umfassend den Dosierungsplan nach Anspruch 8, wobei die Startdosis von 5-[2,4-Difluorphenyl]-salicylsäure in Schritt a) in zwei separaten Teilen als eine erste Teildosis zwischen 20 und 35 mg/kg Körpergewicht des Patienten und dann nach 4 - 8 Stunden als eine zweite Teildosis von 12 - 20 mg/kg Körpergewicht des Patienten verabreicht wird.

10. Salicylate zur Verwendung bei der Behandlung von Krebs, umfassend den Dosierungsplan nach einem der vorstehenden Ansprüche, wobei die mindestens zwei verschiedenen Salicylate in Schritt b) in Teilen zusammen, separat und/oder über den Behandlungstag verteilt verabreicht werden.

11. Salicylate zur Verwendung bei der Behandlung von Krebs, umfassend den Dosierungsplan nach einem der vorstehenden Ansprüche, wobei die Zeit zwischen der letzten Verabreichung einer Dosis an einem beliebigen Behandlungstag und der ersten Verabreichung einer Dosis am nächsten Behandlungstag mindestens 12 Stunden beträgt.

12. Salicylate zur Verwendung bei der Behandlung von Krebs, umfassend den Dosierungsplan nach einem der Ansprüche 1, 4 bis 11, wobei ein Behandlungszyklus **dadurch gekennzeichnet ist, dass**: die Verabreichung von 5-[2,4-Difluorphenyl]-salicylsäure in Schritt a) für vier aufeinanderfolgende Behandlungstage wiederholt wird und die Verabreichung der mindestens zwei verschiedenen Salicylate von Schritt b) für mindestens zwei aufeinanderfolgende Behandlungstage wiederholt wird.

13. Salicylate zur Verwendung bei der Behandlung von Krebs, umfassend den Dosierungsplan nach Anspruch 12, wobei der Behandlungszyklus mindestens dreimal wiederholt wird.

## Revendications

1. Salicylates destinés à être utilisés dans le traitement du cancer, comprenant le schéma posologique suivant :
a) administrer au patient une dose initiale d'acide acétylsalicylique ou d'acide 5-[2,4-difluorophényl]-salicylique le premier Jour du traitement,
b) administrer au patient une seconde dose d'au moins deux salicylates différents choisis parmi : l'acide acétylsalicylique, l'acide 4-aminosalicylique et l'acide 5-[2,4-difluorophényl]-salicylique le Jour suivant du traitement, dans lequel la dose d'acide acétylsalicylique ne dépasse pas 80 mg/kg de poids corporel du patient à l'étape b) lorsque l'acide acétylsalicylique et l'acide 5-[2,4-difluorophényl]-salicylique sont administrés ensemble.

2. Salicylates destinés à être utilisés dans le traitement du cancer, comprenant le schéma posologique selon la revendication 1, dans lequel la seconde dose est administrée à un patient au cours de chacun des trois Jours consécutifs du traitement.

3. Salicylates destinés à être utilisés dans le traitement du cancer, comprenant le schéma posologique selon l'une des revendications précédentes, dans lequel la dose initiale d'acide acétylsalicylique à l'étape a) est comprise entre 40 et 70 mg/kg de poids corporel du patient.

4. Salicylates destinés à être utilisés dans le traitement du cancer, comprenant le schéma posologique selon l'une quelconque des revendications précédentes, dans lequel la dose initiale d'acide 5-[2,4-difluorophényl]-salicylique à l'étape a) est comprise entre 20 et 60 mg/kg de poids corporel du patient.

5. Salicylates destinés à être utilisés dans le traitement du cancer, comprenant le schéma posologique selon l'une quelconque des revendications précédentes, dans lequel la dose d'acide acétylsalicylique à l'étape b) est comprise entre 30 et 120 mg/kg de poids corporel du patient.

6. Salicylates destinés à être utilisés dans le traitement du cancer, comprenant le schéma posologique selon l'une quelconque des revendications précédentes, dans lequel la dose d'acide 4-aminosalicylique à l'étape b) est comprise entre 100 et 300 mg/kg de poids corporel du patient.

7. Salicylates destinés à être utilisés dans le traitement du cancer, comprenant le schéma posologique selon l'une quelconque des revendications précédentes, dans lequel la dose d'acide 5-[2,4-difluorophényl]-salicylique à l'étape b) est comprise entre 20 et 60 mg/kg de poids corporel du patient.

8. Salicylates destinés à être utilisés dans le traitement du cancer, comprenant le schéma posologique selon l'une quelconque des revendications précédentes, dans lequel la dose initiale est administrée en fractions réparties sur le premier Jour du traitement.

9. Salicylates destinés à être utilisés dans le traitement du cancer, comprenant le schéma posologique selon la revendication 8, dans lequel la dose initiale d'acide 5-[2,4-difluorophényl]-salicylique à l'étape a) est administrée en deux fractions séparées, sous forme d'une première fraction de 20 à 35 mg/kg de poids corporel du patient, puis après 4 à 8 heures, sous forme d'une seconde fraction de 12 à 20 mg/kg de poids corporel du patient.

10. Salicylates destinés à être utilisés dans le traitement du cancer, comprenant le schéma posologique selon l'une quelconque des revendications précédentes, dans lequel lesdits au moins deux salicylates différents à l'étape b) sont administrés en fractions, ensemble, séparément et/ou répartis sur la durée du traitement.

11. Salicylates destinés à être utilisés dans le traitement du cancer, comprenant le schéma posologique selon l'une quelconque des revendications précédentes, dans lequel dans lequel un délai d'au moins 12 heures s'écoule entre la dernière administration d'une dose un Jour quelconque du traitement et la première administration d'une dose le Jour suivant du traitement.

12. Salicylates destinés à être utilisés dans le traitement du cancer, comprenant le schéma posologique selon l'une quelconque des revendications 1, 4 à 11, dans lequel un cycle du traitement est **caractérisé par** : l'administration de l'acide 5-[2,4-difluorophényl]-salicylique à l'étape a) est répétée pendant quatre Jours consécutifs du traitement et l'administration dudit au moins deux salicylates différents à l'étape b) est répétée pendant au moins deux Jours consécutifs du traitement.

13. Salicylates destinés à être utilisés dans le traitement du cancer, comprenant le schéma posologique selon la revendication 12, dans lequel le cycle du traitement est répété au moins 3 fois.
